(19)

Europäisches
Patentamt
European
Patent Office
Office européen
des brevets

(11) **EP 3 624 017 A1**

(12) **EUROPEAN PATENT APPLICATION**

(43) Date of publication:
**18.03.2020 Bulletin 2020/12**

(51) Int Cl.:
**G06N 3/04** *(2006.01)*  **G06N 5/04** *(2006.01)*
**G06N 3/08** *(2006.01)*

(21) Application number: **19194591.4**

(22) Date of filing: **30.08.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.09.2018 JP 2018170769**

(71) Applicant: **Hitachi, Ltd.**
**Tokyo 100-8280 (JP)**

(72) Inventors:
• **Shibahara, Takuma**
  **Tokyo, 100-8280 (JP)**
• **Suzuki, Mayumi**
  **Tokyo, 100-8280 (JP)**
• **Yamashita, Yasuho**
  **Tokyo, 100-8280 (JP)**

(74) Representative: **Manitz Finsterwald**
**Patent- und Rechtsanwaltspartnerschaft mbB**
**Martin-Greif-Strasse 1**
**80336 München (DE)**

(54) **TIME SERIES DATA ANALYSIS APPARATUS, TIME SERIES DATA ANALYSIS METHOD AND TIME SERIES DATA ANALYSIS PROGRAM**

(57) Facilitating explanations about analysis of time series data is realized. A time series data analysis apparatus: generates first internal data, based on a first feature data groups, a first internal parameter, and a first learning parameter; transforms a position of first feature data in a feature space, based on the first internal data and a second learning parameter; reallocates the first feature data, based on a first transform result and the first feature data groups; calculates a first predicted value, based on a reallocation result and a third learning parameter; optimizes the first to third learning parameters by statistical gradient, based on a response variable and a first predicted value; generates second internal data, based on second feature data groups, a second internal parameter, and an optimized first learning parameter; transforms a position of the second feature data in a feature space, based on the second internal data and an optimized second learning parameter; and calculates importance data for the second feature data, based on a second transform result and an optimized third learning parameter.

EP 3 624 017 A1

**Description**

CLAIM OF PRIORITY

The present application claims priority from Japanese patent application JP 2018-170769 filed on September 12, 2018, the content of which is hereby incorporated by reference into this application.

BACKGROUND OF THE INVENTION

1. Field of the Invention

The present invention relates to a time series data analysis apparatus, a time series data analysis method, and a time series data analysis program for analyzing time series data.

2. Description of the Related Art

In machine learning that is one of techniques for realizing artificial intelligence (AI), to calculate learning parameters such as weight vectors in perceptrons in such a manner as to minimize an error between a predicted value obtained from feature vectors and an actual value or true value is called learning. Upon completion with a learning process, a new predicted value can be calculated from data not used in the learning, hereinafter the data being referred to as "test data." In the perceptrons, a magnitude of each element value of the weight vectors is used as an importance of a factor contributing to a prediction.
On the other hand, while a neural network including deep learning can realize high prediction accuracy, each element of the feature vectors is subjected to weighted product-sum operation with the other elements whenever passing through a plurality of perceptrons; thus, in principle, it is difficult to grasp the importance of each single element. This is a fatal flaw in a case of using the deep learning in a medical front.
A case in which a medical doctor uses AI in determining whether to discharge a certain patient will be taken by way of example. The AI using the deep learning is unable to output a factor that reached a determination that the certain patient is to be readmitted together with a diagnosis result that the certain patient is "prone to be readmitted" for the certain patient. If the AI can output even the determination factor, the medical doctor can give proper treatment to the patient.
Ribeiro, Marco Tulio, Sameer Singh, and Carlos Guestrin. "Why should I trust you?: Explaining the predictions of any classifier." Proceedings of the 22nd ACM SIGKDD International Conference on Knowledge Discovery and Data Mining. ACM, 2016., hereinafter referred to as a non-patent document 1, is one approach for newly learning linear regression or logistic regression in such a manner as to be capable of explaining an identification result of a machine learning approach such as deep learning without a function to calculate an importance of each feature. Furthermore, the logistic regression is a machine learning model equivalent to the perceptron and most widely used in every field. For example, as disclosed in page 119 of Friedman J, Trevor H, Robert T. The elements of statistical learning. second edition. New York: Springer series in statistics, 2001., the logistic regression has a function to calculate the importance of each feature for entire data samples. Golas, Sara Bersche, et al. "A machine learning model to predict the risk of 30-day readmissions in patients with heart failure: a retrospective analysis of electronic medical records data." BMC medical informatics and decision making 18.1 (2018) : 44. 22 Jun 2018, hereinafter referred to as a non-patent document 3, discloses a machine learning model that configures 3512-dimensional features and that carries out analysis. According to Ashish Vaswani, et al. "Attention is all you need." Advances in Neural Information Processing Systems, 2017., hereinafter referred to as a non-patent document 4, Transformer is one of neural networks capable of handling time series data.
The approach of the non-patent document 1 is inapplicable to a recurrent neural network (RNN) that is the deep learning for time series data. For example, in a case of performing a process without taking into account of time series information, there is a probability of a large divergence between an actually occurring result and a prediction result since the condition of an admitted patient changes on a daily basis.
Furthermore, without making clear the factors influencing past prediction results, the medical doctor is unable to improve future treatment. Moreover, the approach of the non-patent document 1 merely tries to explain the deep learning with the linear regression afterward. Even in a case of trying to explain normal fully connected deep learning, it is not mathematically ensured that the importance of each feature used by the deep learning at the time of prediction can be completely calculated. Providing that the linear regression can completely achieve the same prediction accuracy as that of the deep learning, the deep learning used first is no longer necessary. The approach of the non-patent document 1 has a contradiction in a configuration concept.
The present invention has been achieved in the light of the above problems and an object of the present invention is to realize facilitating explanations about time series data.

SUMMARY OF THE INVENTION

A time series data analysis apparatus according to one aspect of the invention disclosed in the present application is a time series data analysis apparatus accessible to (cabable of accessing) a database, including: a processor that executes a program; and a storage device that stores the program, the database storing a training data set having a predetermined number of first feature data groups in each of which plural pieces of first feature data each containing a plurality of features are present in time series and a predetermined number of response variables each corresponding to each piece of the first feature data in each of the first feature data groups, in which the processor executes: a first generation process generating first internal data based on time of one piece of the first feature data for each piece of the first feature data on a basis of the first feature data groups, a first internal parameter that is at least part of other piece of the first feature data at time before the time of the one piece of the first feature data, and a first learning parameter; a first transform process transforming a position of the one piece of the first feature data in a feature space on a basis of a plurality of first internal data each generated by the first generation process for each piece of the first feature data and a second learning parameter; a reallocation process reallocating each piece of the first feature data into a transform destination position in the feature space on a basis of a first transform result in time series by the first transform process for each piece of the first internal data and the first feature data groups; a first calculation process calculating a first predicted value corresponding to the first feature data groups on a basis of a reallocation result by the reallocation process and a third learning parameter; an optimization process optimizing the first learning parameter, the second learning parameter, and the third learning parameter by statistical gradient on a basis of the response variable and the first predicted value calculated by the first calculation process; a second generation process generating second internal data based on time of one piece of second feature data among plural pieces of the second feature data each containing a plurality of features, the second internal data being generated for each piece of the second feature data on a basis of second feature data groups in each of which the plural pieces of the second feature data each containing the plurality of features are present in time series, a second internal parameter that is at least part of other piece of the second feature data at time before the time of the one piece of the second feature data, and a first learning parameter optimized by the optimization process; a second transform process transforming a position of the one piece of the second feature data in the feature space on a basis of a plurality of second internal data generated by the second generation process for each piece of the second feature data and a second learning parameter optimized by the optimization process; and an importance calculation process calculating importance data indicating an importance of each piece of the second feature data on a basis of a second transform result in time series by the second transform process for each piece of the second internal data and a third learning parameter optimized by the optimization process.

According to a typical embodiment of the present invention, it is possible to realize facilitating explanations about the analysis of time series data. Objects, configurations, and effects other than those described above will be readily apparent from the description of embodiments given below.

BRIEF DESCRIPTION OF THE DRAWINGS

FIG. 1 is an explanatory diagram of a relationship between time series feature vectors and identification boundaries;
FIGS. 2A and 2B are block diagrams depicting an example of a system configuration of a time series data analysis system;
FIG. 3 is an explanatory diagram depicting an example of a structure of a neural network according to the first embodiment;
FIG. 4 is a flowchart depicting an example of learning and prediction processing procedures by a time series data analysis apparatus;
FIG. 5 is an explanatory diagram depicting an example of a neural network setting screen;
FIG. 6 is an explanatory diagram depicting an example of display of an output panel; and
FIG. 7 is chart depicting experimental results by a discriminator based on the non-patent document 4 and by the time series data analysis apparatus according to the first embodiment.

DESCRIPTION OF THE PREFERRED EMBODIMENTS

[First Embodiment]

In a first embodiment, a time series data analysis apparatus for predicting whether a patient admitted due to a heart failure is readmitted at a time of discharge and outputting a factor contributing to the readmission will be described by way of example. The factor output by the time series data analysis apparatus according to the first embodiment enables a medical doctor to give prognostic guidance suited for an individual patient. This can contribute to a prompt recovery of each patient and improving a medical quality, and can lead to cutting back medical costs of a country increasing at an accelerated pace.

<Feature vectors and identification plane in time-space>

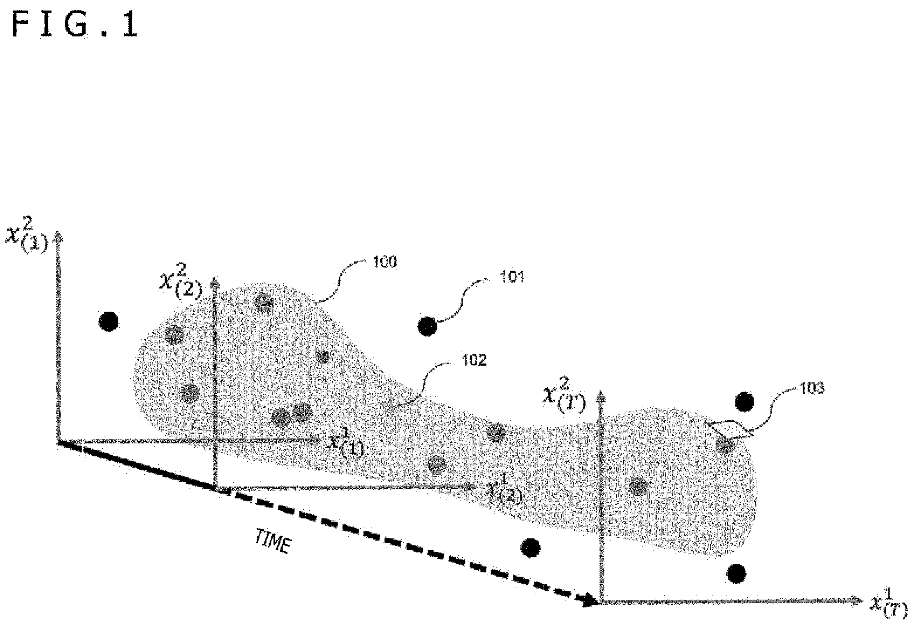

FIG. 1 is an explanatory diagram depicting a relationship between time series feature vectors and identification boundaries. In FIG. 1, a dimension representing time is assumed as one axis and patients are depicted in a feature space laid out by dimensions representing a plurality of other features such as a daily blood pressure. A boundary plane 100 is a true identification boundary plane that separates a patient to be readmitted in the future 101 from a patient not to be readmitted 102. While an RNN has a capability of calculating the boundary plane 100, the boundary plane 100 is generally a complicated curve in high dimensions and is incomprehensive to humans with human capabilities.

On the other hand, even with the complicated high-dimensional curve such as the boundary plane 100, the boundary plane 100 can be often locally regarded as a plane 103. If the local plane 103 can be calculated per patient using a myriad of perceptrons, or logistic regressions, refer to a second embodiment, it is possible to grasp a factor contributing to a prediction as a magnitude of each element value of learning parameters, inclination of the plane, of each of those linear models. The time series data analysis apparatus according to the first embodiment generates a linear model per patient using deep learning capable of processing time series data.

<Example of system configuration>

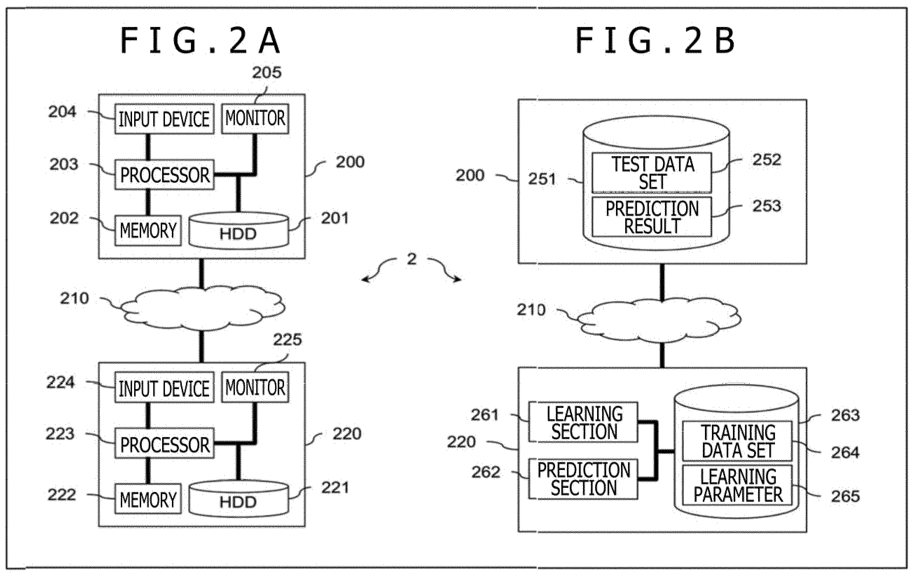

FIGS. 2A and 2B are block diagrams depicting an example of a system configuration of a time series data analysis system. While FIGS. 2A and 2B refer to a server-client type time series data analysis system 2 by way of example, the time series data analysis system may be a stand-alone type time series data analysis system. FIG. 2A is a block diagram depicting an example of a hardware configuration of the time series data analysis system 2, and FIG. 2B is a block diagram depicting an example of a functional configuration of the time series data analysis system 2. In FIGS. 2A and 2B, the same configuration is denoted by the same reference character.

The time series data analysis system 2 is configured such that a client terminal 200 and a time series data analysis apparatus 220 that is a server are communicably connected to each other by a network 210.

In FIG. 2A, the client terminal 200 has a hard disk drive (HDD) 201 that is an auxiliary storage device, a memory 202 that is a main storage device, a processor 203, an input device 204 such as a keyboard and a mouse, and a monitor 205. The time series data analysis apparatus 220 has an HDD 221 that is an auxiliary storage device, a memory 222 that is a main storage device, a processor 223, an input device 224 such as a keyboard and a mouse, and a monitor 225. It is noted that the main storage device, the auxiliary storage device, or a transportable storage medium, which is not depicted, will be generically referred to as "storage device." The storage device stores a neural network 300 and learning parameters thereof.

In FIG. 2B, the client terminal 200 has a client database (DB) 251. The client DB 251 is stored in the storage device such as the HDD 201 or the memory 202. The client DB 251 stores a test data set 252 and a prediction result 253. The test data set 252 is a set of test data. The prediction result 253 is data obtained from a prediction section 262 via the network 210. It is noted that one or more client terminals 200 are present in the case of the server-client type.

The time series data analysis apparatus 220 has a learning section 261, the prediction section 262, and a server database (DB) 263. The learning section 261 is a functional section that outputs learning parameters 265 using the neural network 300.

The prediction section 262 is a functional section that constructs the neural network 300 using the learning parameters 265, that executes a prediction process through test data being given to the neural network 300, and that outputs the prediction result 253 to the client terminal 200. The learning section 261 and the prediction section 262 realize functions thereof by causing the processor 223 to execute a program stored in the storage device such as the HDD 221 or the memory 222.

The server DB 263 stores a training data set 264 and the learning parameters 265. The training data set 264 is a set of training data configured with a combination $\{x_{(t, n)}, Y_{(n)}\}$ of a time series feature vector $x_{(t, n)}$ and a response variable $Y_{(n)}$. $n = \{1, 2, ..., N\}$ and n is, for example, an index for designating patient data. It is assumed in the first embodiment $N = 30,000$.

$t = \{0, 1, ..., T_n-1\}$ and t represents, for example, acquisition time such as the number of weeks from a date of admission, of n-th patient data. Acquisition time intervals are not necessarily fixed intervals for the patient data about one patient. In addition, the acquisition time intervals of the patient data about one patient are not necessary identical to those of the other patient data. In a case in which the acquisition time has different units such as units of seconds, units of minutes, units of hours, units of days, units of months, or units of years, the units are made uniform to a certain unit, a minimum unit, for example, and then the patient data is input.

The time series feature vector $x_{(t, 0)} \in R^D$, where $R^D$ is a D-dimensional real number and D is an integer equal to or greater than 1, is a D-dimensional real-valued vector which contains information such as an age, a gender, administration information at the acquisition time t and a test value at the acquisition time t. According to the non-patent document 3, the machine learning model configures the 3,512-dimensional features and carries out analysis. To the time series data analysis apparatus 220, the time series feature vector $x_{(t, n)}$ can be input similarly to the non-patent document 3.

However, to enhance facilitating understanding the first embodiment, the time series feature vector $x_{(t, n)}$ will be described as {age, gender, white blood cell count [million cells/$\mu$l] per week} (D = three). It is noted that the test data set 252 is a set of test data that are the other time series feature vectors not used as the time series feature vector $x_{(t, n)}$. The other time features that serve as the test data will be denoted by time series feature vector $x'_{(t, n)}$.

The response variable $Y_{(n)}$ takes on a value 0 or 1. In the first embodiment, it means, for example, that if a patient indicated by n-th patient data is readmitted when $Y_{(n)} = 1$, and the patient is not readmitted when $Y_{(n)} = 0$. In the following description, in a case of not distinguishing the index n, n will be often omitted and "time series feature vector $x_{(t)}$" and "response variable Y" are often used. Likewise, n will be omitted for a calculation result using the "time series feature vectors $x_{(t, n)}$ and $x'_{(t, n)}$." Hereinafter, an example of matrix expression of the time series feature vectors $x_{(1)}$ to $x_{(T)}$ with D as three will be described.

[Expression 1]

GENDER (1 FOR MALE AND 0 FOR FEMALE)

AGE      WHITE BLOOD CELL COUNT PER WEEK [million cells/$\mu$l]

$$
\begin{array}{ll}
\vec{x}_{(0)} \Rightarrow \\
\vec{x}_{(1)} \Rightarrow \\
\vdots \\
\vec{x}_{(t)} \Rightarrow \\
\vdots \\
\vec{x}_{(T-2)} \Rightarrow \\
\vec{x}_{(T-1)} \Rightarrow
\end{array}
\begin{bmatrix}
45 & 1 & 6.5 \\
45 & 1 & 6.4 \\
\vdots & \vdots & \vdots \\
45 & 1 & 5.8 \\
\vdots & \vdots & \vdots \\
46 & 1 & 5.3 \\
46 & 1 & 5.2
\end{bmatrix}
\begin{array}{l}
\longleftarrow \text{Week 0 (INITIAL CONSULTATION)} \\
\longleftarrow \text{Week 1} \\
\vdots \\
\longleftarrow \text{Week t} \\
\vdots \\
\longleftarrow \text{Week T-2} \\
\longleftarrow \text{Week T-1}
\end{array}
$$

WHERE $\vec{x}_{(t)}$ IS TIME SERIES FEATURE VECTOR $x_{(t)}$

As described above, a set of the time series feature vectors $x_{(1)}$ to $x_{(T)}$ are expressed as a matrix with T rows and D columns. A matrix that summarizes the time series feature vectors $x_{(1)}$ to $x_{(T)}$ in this way will be denoted by "time series feature vectors x." In this way, T-dimensional features, white blood cell count in the present embodiment, can be summarized into features in one certain dimension, so that calculation efficiency improves.

The learning parameters 265 are output data from the learning section 261 and include learning parameters {RWs, W, w} to be described later. The neural network 300 to which the learning parameters 265 are set will be referred to as "prediction model."

It is noted that the time series data analysis apparatus 220 may be configured with a plurality of apparatuses. For example, a plurality of time series data analysis apparatuses 220 may be present for load distribution. Furthermore, the time series data analysis apparatus 220 may be configured with a plural of apparatuses corresponding to functions. For example, the time series data analysis apparatus 220 may be configured with a first server that includes the learning section 261 and the server DB 263, and a second server that includes the prediction section 262 and the server DB 263. Alternatively, the time series data analysis apparatus 220 may be configured with a first time series data analysis apparatus that includes the learning section 261 and the prediction section 262, and a second time series data analysis apparatus that includes the server DB 263. In another alternative, the time series data analysis apparatus 220 may be configured with a first server that includes the learning section 261, a second time series data analysis apparatus that includes the prediction section 262, and a third time series data analysis apparatus that includes the server DB 263.

<Example of structure of neural network>

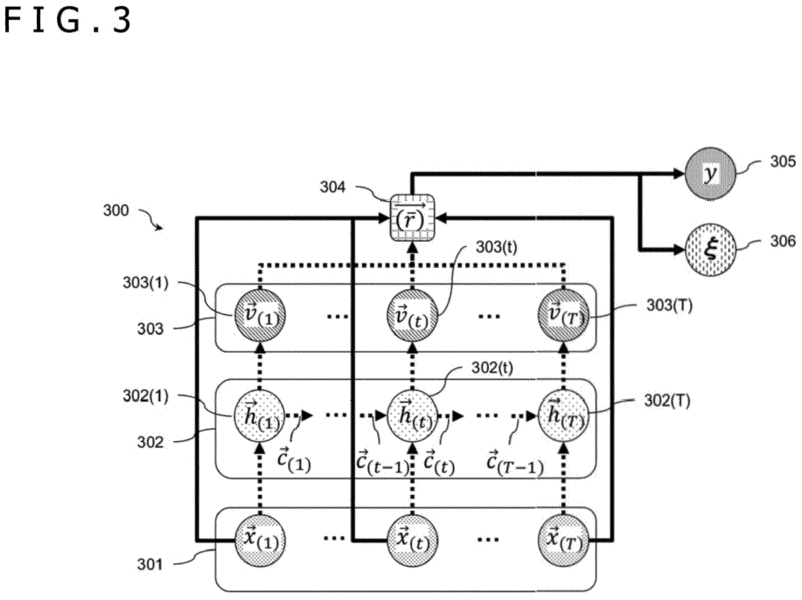

FIG. 3 is an explanatory diagram depicting an example of a configuration of the neural network 300 according to the first embodiment. The neural network 300 is used by the learning section 261 and the prediction section 262. The neural network 300 has a time series data neuron group 302, a transform unit group 303, a reallocation unit 304, a decision unit 305, and an importance unit 306. In addition, a set of the time series feature vectors $x_{(1)}$ to $x_{(T)}$ as input data are depicted as "input unit 301."

The time series data neuron group 302 is a set of T time series data neurons 302(1) to 302 (T) . At a time of learning by the learning section 261, the time series feature vector $x_{(t)}$ that is part of the training data set 264 is input to the time series data neuron 302 (t). As depicted in Equation (1), the time series data neuron 302(t) calculates an internal vector $h_{(t)}$ and an internal state parameter $c_{(t)}$ on the basis of the time series feature vector $x_{(t)}$ and an internal state parameter $c_{(t-1)}$.

[Expression 2]

$$\vec{h}_{(t)}, \vec{c}_{(t)} = RNN\left(\vec{x}_{(t)}, \vec{c}_{(t-1)}\right)$$

$$\text{WHERE } \vec{h}_{(t)} \text{ IS INTERNAL VECTOR } h_{(t)} \in \mathbf{R}^{D'}$$

$$\vec{c}_{(t)} \text{ IS INTERNAL STATE PARAMETER } c_{(t)} \in \mathbf{R}^{D''}$$

$$\cdots \text{Equation (1)}$$

An RNN function on a right side is a function that calculates the internal vector $h_{(t)}$ and the internal state parameter $c_{(t)}$ by recursively inputting features aggregated from the time series feature vectors $x_{(0)}$ to $x_{(t-1)}$ input to a time series data neuron 302 (t-1) before acquisition time (t-1) as well as the time series feature vector $x_{(t)}$ to the time series data neuron 302(t). The RNN function holds the learning parameters RWs that serve as weights.

The learning parameters RWs are a set of the learning parameters RW present in the time series data neuron 302(t) at each acquisition time t. At the time of learning, initial values of the learning parameters RWs are determined at random. The learning parameters RWs are updated whenever the time series feature vector $x_{(t)}$ is input to the time series data neuron 302(t) at the time of learning. The learning parameters RWs are optimized by Equation (6) to be described later.

An internal vector $h_{(t)} \in R^{D'}$, where $R^{D'}$ is a D'-dimensional real number and D' is an integer equal to or greater than 1, is information that reflects an internal state parameter $c_{(t-1)} \in R^{D''}$, where $R^{D''}$ is a D"-dimension real number and D" is an integer equal to or greater than 1, at acquisition time (t-1) just before the acquisition time t in information identified by the time series feature vector $x_{(t)}$. It is noted, however, that the internal state parameter $c_{(0)}$ is a value initialized to zero or a random number. The internal vector $h_{(t)}$ is output to the transform unit group 303 in a rear stage.

On the other hand, the internal state parameter $c_{(t)}$ is output to the time series data neuron 302(t+1) at next acquisition time (t+1) . It is noted, however, that the time series data neuron 302(T) does not output the internal state parameter $c_{(t)}$. The internal state parameter $c_{(t)}$ is a parameter obtained by aggregating information about the features, such as age, gender, and white blood cell count per week, from the time series feature vectors $x_{(1)}$ to $x_{(t-1)}$ before the acquisition time (t-1) just before the acquisition time t by the RNN function. The internal state parameter $c_{(t)}$ is a vector such as encrypted cache information incomprehensible to humans.

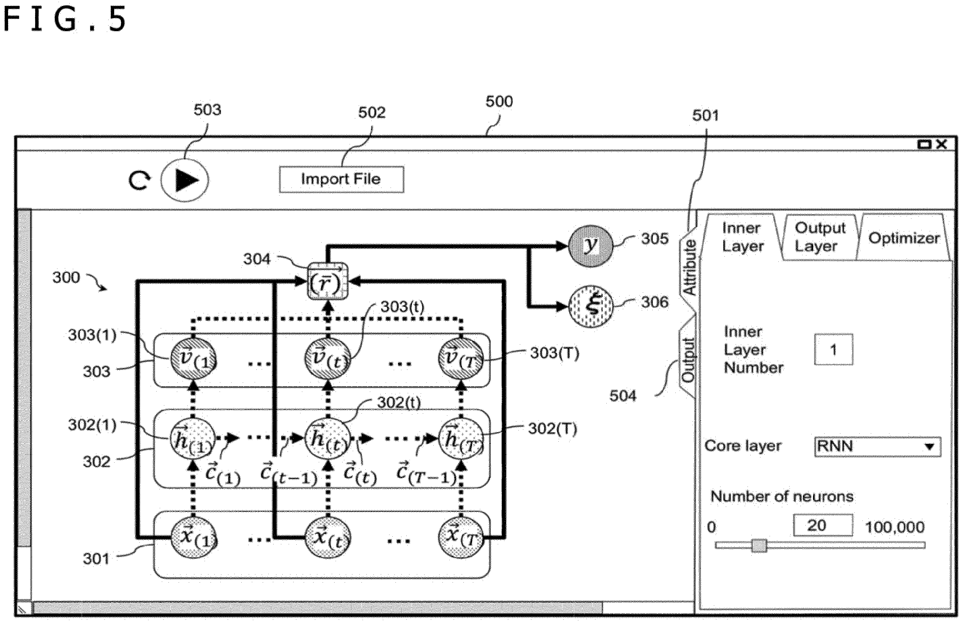

It is noted that an operation by the RNN function in the time series data neuron 302(t) can use an operation by a neural network that can handle time series data such as a long short-term memory (LSTM), a gated recurrent unit (GRU), a Transformer, refer to the non-patent document 4, and a convolutional neural network (CNN). Furthermore, the operation by the RNN function in the time series data neuron 302(t) can be configured as a multi-layered configuration by stacking those time series neural networks. Moreover, a type such as Core layer, and the number of layers such as Inner layer Number, of the time series data neuron 302(t) and the number of dimensions D' of the internal vector can be freely set by user's operation, refer to FIG. 5.

Furthermore, the time series data neuron 302(t) can be executed at a time of prediction by the prediction section 262 similarly to the time of learning. Hereinafter, "'" is added to each information used at the time of prediction like a time series feature vector $x'_{(t)}$. At the time of prediction, time series feature vectors $x'_{(1)}$ to $x'_{(t)}$ that are the test data set 252 are input to the time series data neurons 302(1) to 302(T), respectively.

The time series data neuron 302(t) then gives the time series feature vector $x'_{(t)}$, an internal state parameter $c'_{(t-1)}$, and the learning parameters RWs obtained at the time of learning to the RNN function, and calculates an internal vector $h'_{(t)}$ and an internal state parameter $c'_{(t)}$ by the above Equation (1). The internal vector $h'_{(t)}$ is output to the transform unit group 303 in the later stage.

The transform unit group 303 is a set of T transform units 303 (1) to 303(T). At the time of learning by the learning section 261, the internal vector $h_{(t)}$ is input to the transform unit 303(t) and the transform unit 303(t) calculates a transform vector $v^{\alpha}_{(t)}$ by the following Equation (2). The transform vector $v^{\alpha}_{(t)}$ is output to the reallocation unit 304 in a later stage.

[Expression 3]

$$v^{\alpha}_{(t)} = W^{\alpha}_{\beta} \, h^{\beta}_{(t)}$$

$$\cdots \text{Equation (2)}$$

Equation (2) employs the Einstein summation convention. For example, in $Z^{\alpha} = X^{\alpha}_{\beta} \cdot Y^{\beta}$, it is indicated that X is a matrix with $\alpha$ rows and $\beta$ columns, Y is a matrix with $\beta$ rows, and that Z is a matrix or vector, with $\alpha$ rows and one column. In subsequent equations for explaining operations, the Einstein summation convention is employed. Furthermore, $\alpha$ and $\beta$ will be often omitted.

$W \in R^{D \times D'}$, where $R^{D \times D'}$ is a D $\times$ D' - dimensional real number, is a learning parameter and present per acquisition time t. At the time of learning, an initial value of the learning parameter W is determined at random. The learning parameter W is updated whenever the internal vector $h_{(t)}$ is input to the transform unit 303(t) at the time of learning. A transform vector $v_{(t)}$ is a vector for transforming a position of the time series feature vector $x_{(t)}$ present in a feature space at the acquisition time t into a position that facilitates discriminating a value, that is 0 or 1, of the response variable Y thereof.

Furthermore, the transform unit 303(t) can be executed at the time of prediction by the prediction section 262 similarly to the time of learning. At the time of prediction, internal vectors $h'_{(1)}$ to $h'_{(t)}$ are input to the transform units 303(1) to 303(T), respectively. The transform unit 303(t) then gives the internal vector $h'_{(t)}$ and the learning parameter W optimized by Equation (6) to be described later to Equation (2), and calculates the transform vector $v'_{(t)}$. The transform unit 303(t) outputs the transform vector $v'_{(t)}$ to the reallocation unit 304 in the later stage.

The reallocation unit 304 reallocates the time series feature vector group in the feature space. To describe an operation by the reallocation unit 304, a calculation method of an Hadamard product between the two time series vectors $u_{(t=1,...,T)}$ and $v_{(t=1,...,T)}$ is defined by Equation (3).
[Expression 4]

$$\vec{u}_{(t=1,...,T)} \odot \vec{v}_{(t=1,...,T)} \equiv \left\{ \begin{bmatrix} u_{1,(1)} v_{1,(1)} \\ \vdots \\ u_{D,(1)} v_{D,(1)} \end{bmatrix}, \quad \cdots, \quad \begin{bmatrix} u_{1,(T)} v_{1,(T)} \\ \vdots \\ u_{D,(T)} v_{D,(T)} \end{bmatrix} \right\}$$

$$\text{where} \quad \vec{u}_{(t=1,...,T)} = \left\{ \begin{bmatrix} u_{1,(1)} \\ \vdots \\ u_{D,(1)} \end{bmatrix}, \quad \cdots, \quad \begin{bmatrix} u_{1,(T)} \\ \vdots \\ u_{D,(T)} \end{bmatrix} \right\}$$

$$\text{and}$$

$$\vec{v}_{(t=1,...,T)} = \left\{ \begin{bmatrix} v_{1,(1)} \\ \vdots \\ v_{D,(1)} \end{bmatrix}, \quad \cdots, \quad \begin{bmatrix} v_{1,(T)} \\ \vdots \\ v_{D,(T)} \end{bmatrix} \right\}$$

$$\text{WHERE } \vec{u}_{(t=1,...,T)} \quad \text{IS TIME SERIES VECTOR } u_{(t=1,...,T)}$$

$$\vec{v}_{(t=1,...,T)} \quad \text{IS TIME SERIES VECTOR } v_{(t=1,...,T)}$$

$$\cdots \text{Equation (3)}$$

At the time of learning by the learning section 261, the time series feature vectors $x_{(1)}$ to $x_{(T)}$ and the transform vectors $v_{(1)}$ to $v_{(T)}$ are input to the reallocation unit 304, and the reallocation unit 304 calculates a reallocation vector $R^{\alpha} \in R^{D}$ by the following Equation (4). The reallocation unit 304 outputs the reallocation vector $R^{\alpha}$ to the decision unit 305 and the importance unit 306 in later stages. It is noted that $r^{\alpha}_{(t)}$ on a right side is a reallocation vector at the acquisition time t and is an Hadamard product between the transform vector $v_{(t)}$ and the time series feature vector $x_{(t)}$. The reallocation vector $R^{\alpha}$ is an average value of reallocation vectors $r^{\alpha}_{(1)}$ to $r^{\alpha}_{(T)}$.
[Expression 5]

$$R^\alpha = \frac{1}{T-1}\sum_t v^\alpha_{(t)} \odot x^\alpha_{(t)} = \frac{1}{T-1}\sum_t r^\alpha_{(t)}$$

$$\cdots \text{Equation (4)}$$

Furthermore, the reallocation unit 304 can be executed at the time of prediction by the prediction section 262 similarly to the time of learning. At the time of prediction, the time series feature vectors x'$_{(1)}$ to x'$_{(T)}$ and transform vectors v'$_{(t)}$ to v'$_{(t)}$ are input to the reallocation unit 304. The reallocation unit 304 then gives the time series feature vectors x'$_{(1)}$ to x'$_{(T)}$ and the transform vectors v'$_{(t)}$ to v'$_{(t)}$ to Equation (4), and calculates the reallocation vector R'$^\alpha \in$ R$^D$. The reallocation unit 304 outputs the reallocation vector R'$^\alpha$ to the decision unit 305 and the importance unit 306 in the later stages.

At the time of learning by the learning section 261, the decision unit 305 calculates a predicted value y$_{(n)}$ corresponding to the response variable Y$_{(n)}$ by the following Equation (5).

[Expression 6]

$$y = \sigma(w_\alpha R^\alpha)$$

$$\cdots \text{Equation (5)}$$

In Equation (5), $\sigma$ is a sigmoid function, w $\in$ R$^D$ is a learning parameter, and the predicted value y$_{(n)}$ is a readmission probability value. At the time of learning, an initial value of the learning parameter w is determined at random. The learning parameter w is updated whenever the reallocation vector R$^\alpha$ is input to the reallocation unit 304 at the time of learning. It is noted that in a case of solving identification tasks of a plurality of classes, a softmax function is employed as an alternative to the sigmoid function $\sigma$.

Moreover, the learning section 261 gives the response variable Y$_{(n)}$ and the predicted value y$_{(n)}$ to the following Expression (6) using statistical gradient, and calculates {RWs, W, w} that are the learning parameters 265 in such a manner as to minimize a cross entropy therefor. {RWs, W, w} are thereby optimized. The learning section 261 stores the optimized {RWs, W, w} in the server DB 263. By applying the optimized {RWs, W, w} to the neural network 300, the learning model is generated.

[Expression 7]

$$argmin_{\{RWs,W,w\}} \sum_n -(Y_{(n)} \log(y_{(n)}) + (1 - Y_{(n)})\log(1 - y_{(n)}))$$

$$\cdots \text{Equation (6)}$$

At the time of prediction by the prediction section 262, the importance unit 306 calculates importance vectors. To describe an operation by the importance unit 306, a calculation method of an Hadamard product between the vector w and the time series vector u$_{(t=1,...,T)}$ is defined by Expression (7) .

[Expression 8]

$$\vec{w} \odot \vec{u}_{(t=1,\ldots,T)} \equiv \left\{ \begin{bmatrix} w_1 u_{1,(1)} \\ \vdots \\ w_D u_{D,(1)} \end{bmatrix}, \quad \cdots, \quad \begin{bmatrix} w_1 u_{1,(T)} \\ \vdots \\ w_D u_{D,(T)} \end{bmatrix} \right\}$$

$$\text{where} \quad \vec{w} = \begin{bmatrix} w_1 \\ \vdots \\ w_D \end{bmatrix}$$

$$\text{and}$$

$$\vec{u}_{(t=1,\ldots,T)} = \left\{ \begin{bmatrix} u_{1,(1)} \\ \vdots \\ u_{D,(1)} \end{bmatrix}, \quad \cdots, \quad \begin{bmatrix} u_{1,(T)} \\ \vdots \\ u_{D,(T)} \end{bmatrix} \right\}$$

$$\text{WHERE } \vec{w}_{(t=1,\ldots,T)} \text{ IS TIME SERIES VECTOR } w_{(t=1,\ldots,T)}$$
$$\vec{u}_{(t=1,\ldots,T)} \text{ IS TIME SERIES VECTOR } u_{(t=1,\ldots,T)}$$

$$\cdots \text{ Equation (7)}$$

The optimized learning parameter w and the transform vector $v'_{(t)}$ are input to the importance unit 306, and the importance unit 306 calculates an importance vector $\xi_{\alpha,(t)}(x')$ of the time series feature vector x' by the following Equation (8) reflective of Expression (7). Each element of the importance vector $\xi_{\alpha,(t)}(x')$ represents an importance with which the element contributes to a readmission prediction in n-th patient data, time series feature vector x', within the test data set 252 at certain acquisition time t. The prediction section 262 stores the importance vector $\xi_{\alpha,(t)}(x')$ in the client DB 251 as the prediction result 253. The prediction section 262 executes a logistic regression at each acquisition time t by the following Equation (8).
[Expression 9]

$$\xi_{\alpha,(t)}(\vec{x}') = w_\alpha \odot v'_{\alpha,(t)}$$

$$\text{WHERE } \vec{x}'_{(t)} \quad \text{IS TIME SERIES FEATURE VECTOR } x'_{(t)}$$

$$\cdots \text{ Equation (8)}$$

In Equation (8), the transform vector $v'_{(t)}$ is calculated by an inner product between the optimized learning parameter W and the internal vector $h'_{(t)}$ as illustrated by Equation (2). The internal vector $h'_{(t)}$ is obtained by giving the time series feature vector $x'_{(t)}$ and the internal state parameter $c_{(t-1)}$ at time just before the acquisition time t to the RNN function to which the optimized learning parameters RWs are applied as illustrated by the above Equation (1).
In other words, the features aggregated from the time series feature vectors $x'_{(0)}$ to $x'_{(t-1)}$ input to the time series data neuron 302 (t-1) before the acquisition time (t-1) as well as the time series feature vector $x'_{(t)}$ are recursively input to the RNN function, and the RNN function calculates the internal vector $h'_{(t)}$ and the internal state parameter $c'_{(t)}$.
At the time of prediction by the prediction section 262, the decision unit 305 calculates an unknown predicted value $y'_{(n)}$ for the time series feature vector x' by the following Equation (9) using the importance vector $\xi_{\alpha,(t)}(x')$ obtained by Expression (7).
[Expression 10]

$$y'_{(n)} = \sigma\left(\frac{1}{T_n - 1}\sum_t \xi_{\alpha,(t)}(\vec{x}'_{(n)})x'^{\alpha}_{(t,n)}\right)$$

$$\cdots \text{ Equation (9)}$$

In Equation (9), the importance vector $\xi_{\alpha,(t)}(x')$ calculated by the Hadamard product between the optimized learning parameter w and the transform vector $v'_{(t)}$ is employed. Therefore, the decision unit 305 gives the time series feature vectors $x'_{(1)}$ to $x'_{(T)}$ to Equation (9), thereby calculating the unknown predicted value $y'_{(n)}$ for the time series feature vectors $x'_{(1)}$ to $x'_{(T)}$ by the neural network 300 reflective of the optimized learning parameters 265 {RWs, W, s}.
In Equation (9), an importance vector $\xi_{\alpha,(t)}(x'_{(n)})$ corresponds to a parameter of the local plane 103 for identifying the time series feature vector $x'_{(t,n)}$. The prediction section 262 stores the predicted value $y'_{(n)}$ in the client DB 251 as the prediction result 253 while, for example, associating the predicted value with $y'_{(n)}$ with the importance vector $\xi_{\alpha, (t)}(x'_{(n)})$.

<Example of learning and prediction processing procedures>

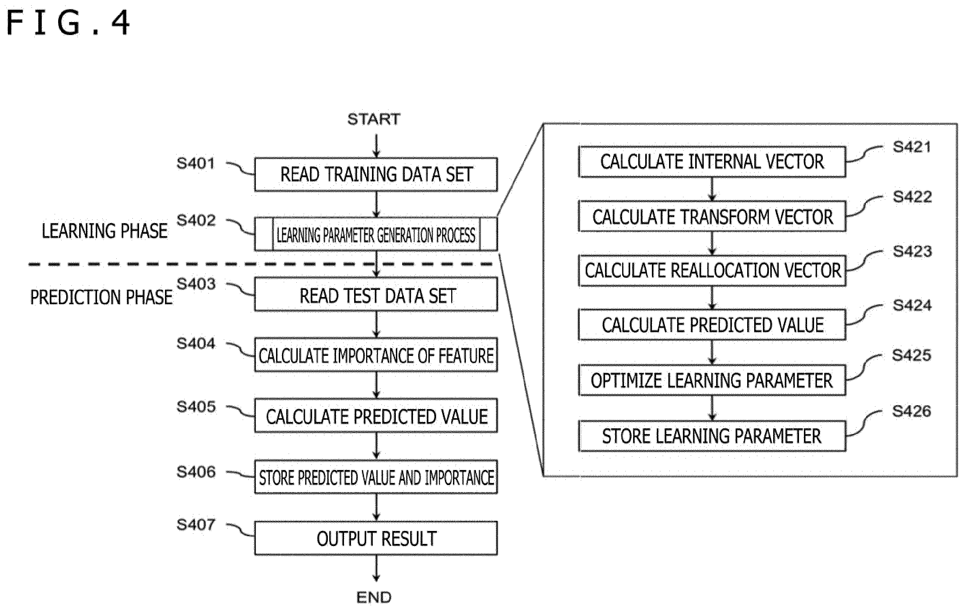

FIG. 4 is a flowchart depicting an example of learning and prediction processing procedures by the time series data analysis apparatus. Steps S401 and S402 correspond to a learning phase executed by the learning section 261, while Steps S403 to S407 correspond to a prediction phase executed by the prediction section 262. First, the learning section 261 reads the training data set 264 from the server DB 263 (Step S401), and executes a learning parameter generation process (Step S402).

In executing the learning parameter generation process (Step S402), the learning section 261 gives the time series feature vector $x_{(t, n)}$ that is part of the training data set 264 to the neural network 300, thereby calculating the internal vector $h_{(t)}$ and the internal state parameter $c_{(t)}$ by Equation (1) as described above (Step S421).

Next, the learning section 261 calculates the transform vector $v^{\alpha}_{(t)}$ by Equation (2) (Step S422). Next, the learning section 261 calculates the reallocation vector $R^{\alpha}$ (Step S423) by the above described Equation (4). The learning section 261 then calculates the predicted value $y_{(n)}$ corresponding to the response variable $Y_{(n)}$ by Equation (5) (Step S424).

The learning section 261 then gives the predicted value $y_{(n)}$ calculated by the above described Equation (5) and the response variable $Y_{(n)}$ that is part of the training data set 264 to Expression (6), thereby optimizing the {RWs, W, w} that are the learning parameters 265 (Step S425). The optimized learning parameters {RWs, W, w} are thereby generated. The learning section 261 then stores the generated learning parameters 265 in the server DB 263 (Step S426).

Next, the prediction section 262 reads the time series feature vector $x'_{(t, n)}$ that is the test data set 252 from the client DB 251 (Step S403). The prediction section 262 then calculates the importance of each feature (Step S404). Specifically, the prediction section 262 causes, for example, the importance unit 306 to give the optimized learning parameter w and the transform vector $v'_{(t)}$ to Equation (8), thereby calculating the importance vector $\xi_{\alpha, (t)}(x')$ of the time series feature vector x'.

Next, the prediction section 262 causes the decision unit 305 to give the time series feature vector $x'_{(t, n)}$ and the importance vector $\xi_{\alpha, (t)}(x')$ obtained by Equation (8) to Equation (9), thereby calculating the unknown predicted value y'(n) (Step S405). The prediction section 262 then stores a combination of the calculated predicted value $y'_{(n)}$ and the calculated importance vector $\xi_{\alpha, (t)}(x')$ in the client DB 251 as the prediction result 253 (Step S406). Subsequently, the client terminal 200 displays the prediction result 253 on the monitor 205 (Step S407).

It is noted that the time series data analysis apparatus 220 may store the prediction result 253 in the server DB 263 in Step S406. Furthermore, the time series data analysis apparatus 220 may transmit the prediction result 253 to the client terminal 200 to cause the client terminal 200 to display the prediction result 253 on the monitor 205 in Step S407.

<Example of neural network setting screen>

FIG. 5 is an explanatory diagram depicting an example of a neural network setting screen. The neural network setting screen 500 can be displayed on the monitors 205 and 225. In a case of displaying the setting screen 500 on the monitor 205, the client terminal 200 can set the neural network. In a case of displaying the setting screen 500 on the monitor 225, the time series data analysis apparatus 220 can set the neural network.

A user edits detailed setting of the neural network on an attribute panel 501. On the attribute panel 501, "Inner Layer Number" indicates the number of layers of the time series data neuron group 302. In the neural network 300 depicted in FIG. 5, the number of layers of the time series data neuron group 302 is one. Whenever the number of layers increases, one time series data neuron group 302 is added in a longitudinal direction between the input unit 301 and the transform unit group 303.

On the attribute panel 501, "Core layer" indicates the type of the time series data neuron group 302. "RNN" is set in FIG. 5. Furthermore, "Number of neurons" indicates the number of dimensions D' of the internal vector.

By depressing an Import File button 502, the user selects a file to be analyzed from a file group list. The training data set 264 is thereby set to the server DB 263 and the test data set 252 is thereby set to the client DB 251. By user's depressing a start operation button 503, the learning process and the prediction process depicted in FIG. 4 are executed. An output panel 504 displays the prediction result 253 of the prediction process depicted in FIG. 4.

<Example of display of output panel 504>

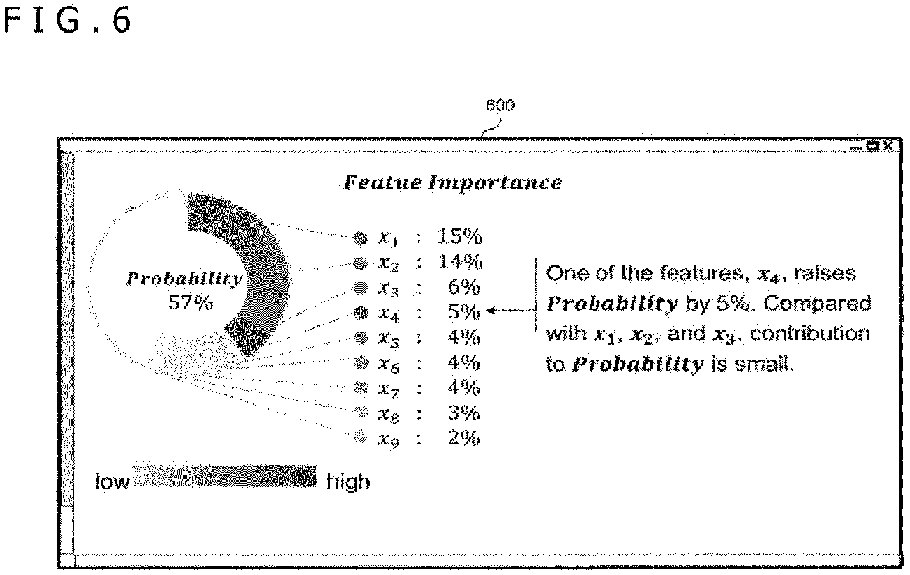

FIG. 6 is an explanatory diagram depicting an example of display of the output panel 504. The prediction result 253 is displayed on a display screen 600 of the output panel 504. In FIG. 6, "57%" in "Probability" indicates the predicted value $y'_{(n)}$. $x_1$ to $x_9$ are nine-dimensional features with D = nine configuring the time series feature vectors $x'_{(t, n)}$ that are the test data set 252. Percentages of the features $x_1$ to $x_9$ are each a numeric value obtained by normalizing a value of the importance vector $\xi_{\alpha, (t)}(x')$ and expressing the normalized value by a percentage.

<Experimental example>

An example of predicting a state of test value on a next day from patient's biochemical test value information on a daily basis is supposed. It is assumed that an operation check of the time series data analysis apparatus 220 according to the first embodiment is carried out using simulation data. The simulation data is a time series feature vector when it is defined that the number of patient data N is 384 samples (N = 384), the number of dimensions D is 1129 (D = 1129), a maximum value T of the patient data acquisition time t such as the number of weeks from the date of admission is 10 (T = 10) .

While the test value information is normally, approximately 100 dimensions at most, the number of dimensions was set to about ten times as large as the normal number to confirm a prediction performance. Features in the dimensions are correlated to one another, and the first-dimensional feature is an average value of the other features. Furthermore, the response variable Y was generated as 1 if the first-dimensional feature at acquisition time T was higher than the average value of the first-dimensional features from acquisition time t = 0, ..., and T-1, and as 0 if the first-dimensional feature at the acquisition time T was lower than the average value.

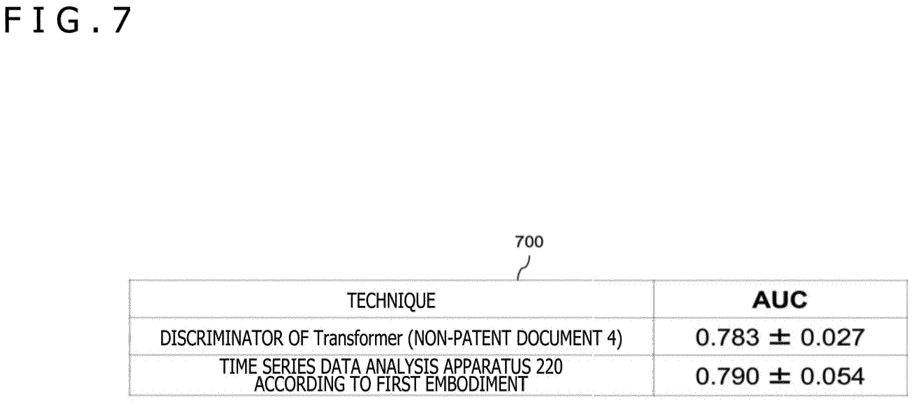

FIG. 7 is a chart depicting experimental results of the discriminator based on the Transformer, refer to the non-patent document 4, and the time series data analysis apparatus 220 according to the first embodiment. In a chart 700, an experiment was conducted using 10-fold cross validation at a measure of area under curve (AUC).

The discriminator based on Transformer, refer to the non-patent document 4, had 0.783 $\pm$ 0.027 and the time series data analysis apparatus 220 according to the first embodiment had 0.790 $\pm$ 0.054. The time series data analysis apparatus 220 according to the first embodiment achieved a performance exceeding that of the Transformer, refer to the non-patent document 4.

In this way, according to the first embodiment, even in the case of the patient's time series data, the importance of each feature at every acquisition time can be calculated for an individual patient. The time series data analysis apparatus 220 according to the first embodiment can, therefore, realize facilitating explanations with high accuracy and with high efficiency.

[Second Embodiment]

In a second embodiment, the time series data analysis apparatus 220 capable of handling an approach classified into a regression will be described. In the second embodiment, an example of predicting a blood pressure of a patient on a next day of admission due to a heart failure and outputting a factor contributing to the blood pressure will be described. The factor output by the time series data analysis apparatus 220 according to the second embodiment enables the medical doctor to give prognostic guidance suited for the individual patient. This can contribute to the prompt recovery of each patient and lead to cutting back medical costs and health costs of a country. Since the second embodiment is described while attention is paid to differences of the second embodiment from the first embodiment, the same content as those in the first embodiment is denoted by the same reference character and explanation thereof will be often omitted.

The training data set 264 is a set of training data configured with a combination $\{x_{(t, 0)}, Y_{(n)}\}$ of the time series feature vector $x_{(t, n)}$ and the response variable $Y_{(n)}$. n = {1, 2, ..., N} and n is, for example, the index for designating patient data. It is assumed in the first embodiment N = 30, 000. t = {0, 1, ..., $T_n$-1} and t represents, for example, acquisition time such as the number of weeks from a date of admission, of n-th patient data. Acquisition time intervals are not necessarily fixed intervals for the patient data about one patient. In addition, the acquisition time intervals of the patient data about one patient are not necessary identical to those of the other patient data.

The time series feature vectors $X_{(t, n)} \in R^D$, where $R^D$ is a D-dimensional real number and D is an integer equal to or greater than 1, are each a D-dimensional real-valued vector which contains information such as the age, the gender, administration information at the time t, and a test value at the time t. According to the non-patent document 3, the machine learning configures features in D = 3, 512 dimensions and carries out analysis. The time series feature vector $x_{(t, n)}$ in the second embodiment can be input to the time series data analysis apparatus 220 similarly to the non-patent document 3.

However, to enhance facilitating understanding the second embodiment, the time series feature vector $x_{(t, n)}$ will be described as {age, gender, blood pressure [mmHg] per week} (D = three dimensions) . The response variable $Y_{(T, n)}$ indicates a blood pressure during a T-th week. It is noted that the test data set 252 is a set of test data that are the other time series feature vectors not used as the time series feature vector $x_{(t, n)}$. The other time series features that serve as the test data will be denoted by time series feature vector $x'_{(t, n)}$.

While the time series data analysis apparatus 220 according to the second embodiment executes similar calculation to that in the first embodiment in the learning phase and the prediction phase, the decision unit 305 in the second embodiment calculates the following Equation (10) as an alternative to Equation (5) and obtains a predicted value y. The predicted value y indicates a patient's blood pressure.
[Expression 11]

$$y = w_\alpha R^\alpha$$

$\cdots$ Equation (10)

Moreover, the learning section 261 gives the response variable $Y_{(n)}$ and the predicted value $y_{(n)}$ to the following Expression (11) as an alternative to Expression (6) using statistical gradient, and calculates {RWs, W, w} that are the learning parameters 265 in such a manner as to minimize the cross entropy therefor. {RWs, W, w} are thereby optimized. The learning section 261 stores the optimized {RWs, W, w} in the server DB 263.
[Expression 12]

$$argmin_{\{RWs,W,w\}} \sum_{n=1}^{N} \left( Y_{(n)} - y_{(n)} \right)^2$$

$\cdots$ Equation (11)

[1] In this way, the time series data analysis apparatus 220 according to the first and second embodiments described above is accessible to (accesses) the server DB 263. The server DB 263 stores the training data set 264 having a predetermined number N of first feature data groups, $x_{(1)}$ to $x_{(T)}$, in each of which the first feature data $x_{(t)}$ each containing a plurality D of features is present in time series, t = 0 to T-1, and the predetermined number N of response variables Y each corresponding to each first feature data in the first feature data groups.

The time series data analysis apparatus 220 executes a first generation process, using the time series data neuron group 302 and Equation (1) in Step S421, for generating first internal data $h_{(t)}$ based on time of the first feature data per first feature data on the basis of the first feature data groups, a first internal parameter $c_{(t-1)}$ that is at least part of other first feature data at time before the time of the first feature data, and the first learning parameter RW.

The time series data analysis apparatus 220 executes a first transform process, using the transform unit group 303 and Equation (2) in Step S422, for transforming a position of the first feature data in the feature space on the basis of a plurality of first internal data $h_{(t)}$ each generated by the first generation process per first feature data and the second learning parameter W.

The time series data analysis apparatus 220 executes a reallocation process, using the reallocation unit 304 and Equation (4) in Step S423, for reallocating each piece of the first feature data into a transform destination position in the feature space on the basis of a first transform result, the transform vector $v_{(t)}$, in time series by the first transform process per first internal data and the first feature data groups, $x_{(1)}$ to $x_{(T)}$.

The time series data analysis apparatus 220 executes a first calculation process, using the decision unit 305 and Equation (5) in Step S424, for calculating the first predicted value y corresponding to the first feature data groups on the basis of a reallocation result, reallocation vector R, by the reallocation process and the third learning parameter w.

The time series data analysis apparatus 220 executes an optimization process, using Expression (6) in Step S425, for optimizing the first learning parameter RW, the second learning parameter W, and the third learning parameter w by statistical gradient on the basis of the response variable Y and the first predicted value y calculated by the first calculation process.

The time series data analysis apparatus 220 executes a second generation process, using the time series data neuron group 302 and Equation (1) in Step S404, for generating second internal data $h'_{(t)}$ based on time of second feature data each containing a plurality D of features per second feature data on the basis of second feature data groups $x'_{(1)}$ to $x'_{(t)}$ in each of which the second feature data each containing the plurality D of features is present in time series t = 0 to T-1, the second internal parameter $c'_{(t-1)}$ that is at least part of other second feature data at time before the time of the second feature data, and the first learning parameter RW optimized by the optimization process.

The time series data analysis apparatus 220 executes a second transform process, using the transform unit group 303 and Equation (2) in Step S404, for transforming a position of the second feature data in the feature space on the basis of a plurality of second internal data $h'_{(t)}$ generated by the second generation process per second feature data and the second learning parameter W optimized by the optimization process.

The time series data analysis apparatus 220 executes an importance calculation process, using the importance unit 306 and Equation (8) in Step S404, for calculating importance data $\xi$ indicating an importance of each piece of the second feature data on the basis of a second transform result, transform vector $v'_{(t)}$, in time series by the second transform process per second internal data and the third learning parameter w optimized by the optimization process.

It is thereby possible to identify the importance of each second feature data. It is, therefore, possible to give an explanation as to what feature is how important at what timing. In this way, it is possible to realize facilitating explanations. Furthermore, even if the boundary plane 100 that can be identified in the feature space is the complicated and high-dimensional curve, locally regarding the boundary plane 100 as the plane 103 makes it possible to realize facilitating explanations with high accuracy and with high efficiency.

[2] The time series data analysis apparatus 220 according to [1] may execute the first generation process and the second generation process using a recurrent neural network.

The recurrent neural network can thereby calculate the complicated and high-dimensional boundary plane 100 that is normally incomprehensive to humans with human capabilities, and realize facilitating explanations with high accuracy and with high efficiency.

[3] The time series data analysis apparatus 220 according to [1], may execute the first generation process and the second generation process using a convolutional neural network.

It is thereby possible to identify the importance of each second feature data while making use of an existing neural network. This can, therefore, facilitate constructing the time series data analysis apparatus 220.

[4] The time series data analysis apparatus 220 according to [1] may execute the first calculation process as an identification operation of the first feature data groups.

It is thereby possible to classify test data in the light of time series of the test data. For example, the prediction accuracy for whether or not the patient identified by the test data is readmitted or for when the patient is readmitted can improve, and the medical doctor can give prognostic guidance suited for an individual patient.

[5] The time series data analysis apparatus 220 according to [1] may execute the first calculation process as a regression operation of the first feature data groups.

It is thereby possible to predict a temporal change in the test data. For example, the prediction accuracy for what value the blood pressure of the patient identified by the test data is at what timing in the future improves, and the medical doctor can give prognostic guidance suited for an individual patient.

[6] The time series data analysis apparatus 220 according to [1] may execute a second calculation process, using the decision unit 305 and Equation (9), for calculating the second predicted value y' corresponding to the second feature data groups on the basis of the importance data $\xi$ calculated by the importance calculation process and the second feature data groups.

It is thereby possible to relatively specify what the importance of a factor of each second feature data in the second feature data groups contributing to the prediction is. Therefore, the time series data analysis apparatus 220 can predict approximately when such a prediction result, second predicted value, caused by what second feature data occurs. For example, in a case in which a prediction result of the readmission appears for the first time at timing at which the importance of the white blood cell count is higher than those of the other second feature data, it is recognized that the feature contributing to the readmission is the white blood cell count. The medical doctor can, therefore, give prognostic guidance and treatment beforehand in such a manner that the white blood cell count falls by the timing. Moreover, using the importance data makes it possible to improve operation efficiency of the second calculation process.

[7] The time series data analysis apparatus 220 according to [6] may execute an output process outputting the second feature data and the importance data to be associated with each other. The medical doctor can thereby confirm what second feature data influences the second predicted value.

The present invention is not limited to the embodiments described above but encompasses various modifications and equivalent configurations within the meaning of the accompanying claims. For example, the above-mentioned embodiments have been described in detail for describing the present invention so that the present invention is easy to understand, and the present invention is not always limited to the embodiments having all the described configurations. Furthermore, a part of the configurations of a certain embodiment may be replaced by configurations of another embodiment. Moreover, the configurations of another embodiment may be added to the configurations of the certain embodiment. Further, for part of the configurations of each embodiment, addition, deletion, or replacement may be made of the other configurations.

Moreover, a part of or all of those including the configurations, the functions, the processing sections, processing means, and the like described above may be realized by hardware by being designed, for example, as an integrated circuit, or may be realized by software by causing the processor to interpret and execute programs that realize the functions.

Information in programs, tables, files, and the like for realizing the functions can be stored in a memory device such as a memory, a hard disc, or a solid state drive (SSD), or in a recording medium such as an integrated circuit (IC) card, an SD card, or a digital versatile disc (DVD).

Furthermore, control lines or information lines considered to be necessary for the description are illustrated and all the control lines or the information lines necessary for implementation are not always illustrated. In actuality, it may be contemplated that almost all the configurations are mutually connected.

Features described in this specification and the claims or shown in a figure shall be deemed combinable with and amongst each other also if their combination is not expressly described, to the extent that this combination is technically feasible. Features described in a certain context, embodiment, figure or claim shall be deemed separable from this claim, context, embodiment or figure and shall be deemed combinable with every other figure, claim, context or embodiment, to the extent that it is technically feasible. Embodiments shall not be understood as being necessarily meant exclusive against each other. Descriptions of a method or procedure or a method step or a procedural step shall be understood also as description of means and/or possibly program instructions of executable code on a data carrier adapted for implementing the method or procedure or method step or procedural step, and vice versa.

**Claims**

1.  A time series data analysis apparatus capable of accessing a database, comprising:

    a processor that executes a program; and
    a storage device that stores the program,
    the database storing a training data set having a predetermined number of first feature data groups in each of which plural pieces of first feature data each containing a plurality of features are present in time series and a predetermined number of response variables each corresponding to each piece of the first feature data in each of the first feature data groups, wherein
    the processor executes:

       a first generation process generating first internal data based on time of one piece of the first feature data for each piece of the first feature data on a basis of the first feature data groups, a first internal parameter that is at least part of other piece of the first feature data at time before the time of the one piece of the first feature data, and a first learning parameter;
       a first transform process transforming a position of the one piece of the first feature data in a feature space on a basis of a plurality of first internal data each generated by the first generation process for each piece of the first feature data and a second learning parameter;
       a reallocation process reallocating each piece of the first feature data into a transform destination position in the feature space on a basis of a first transform result in time series by the first transform process for each piece of the first internal data and the first feature data groups;
       a first calculation process calculating a first predicted value corresponding to the first feature data groups on a basis of a reallocation result by the reallocation process and a third learning parameter;
       an optimization process optimizing the first learning parameter, the second learning parameter, and the third learning parameter by statistical gradient on a basis of the response variable and the first predicted value calculated by the first calculation process;
       a second generation process generating second internal data based on time of one piece of second feature data among plural pieces of the second feature data each containing a plurality of features, the second internal data being generated for each piece of the second feature data on a basis of second feature data groups in each of which the plural pieces of the second feature data each containing the plurality of features are present in time series, a second internal parameter that is at least part of other piece of the second feature data at time before the time of the one piece of the second feature data, and a first learning parameter optimized by the optimization process;
       a second transform process transforming a position of the one piece of the second feature data in the feature space on a basis of a plurality of second internal data generated by the second generation process for each piece of the second feature data and a second learning parameter optimized by the optimization process; and
       an importance calculation process calculating importance data indicating an importance of each piece of the second feature data on a basis of a second transform result in time series by the second transform process for each piece of the second internal data and a third learning parameter optimized by the optimization process.

2.  The time series data analysis apparatus according to claim 1, wherein
    the processor executes the first generation process and the second generation process using a recurrent neural network.

**3.** The time series data analysis apparatus according to claim 1, wherein
the processor executes
the first generation process and the second generation process using a convolutional neural network.

**4.** The time series data analysis apparatus according to claim 1, wherein
the processor executes
the first calculation process as an identification operation of the first feature data groups.

**5.** The time series data analysis apparatus according to claim 1, wherein
the processor executes
the first calculation process as a regression operation of the first feature data groups.

**6.** The time series data analysis apparatus according to claim 1, wherein
the processor executes
a second calculation process calculating a second predicted value corresponding to the second feature data groups on a basis of the importance data calculated by the importance calculation process and the second feature data groups.

**7.** The time series data analysis apparatus according to claim 6, wherein
the processor executes
an output process outputting the second feature data and the importance data to be associated with each other.

**8.** A time series data analysis method by a time series data analysis apparatus capable of accessing a database, the time series data analysis apparatus including a processor that executes a program; and a storage device that stores the program, the database storing a training data set having a predetermined number of first feature data groups in each of which plural pieces of first feature data each containing a plurality of features are present in time series and a predetermined number of response variables each corresponding to each piece of the first feature data in the first feature data groups,
the method allowing the processor to execute the processes comprising:

   a first generation process generating first internal data based on time of one piece of the first feature data for each piece of the first feature data on a basis of the first feature data groups, a first internal parameter that is at least part of other piece of the first feature data at time before the time of the one piece of the first feature data, and a first learning parameter;
   a first transform process transforming a position of the one piece of the first feature data in a feature space on a basis of a plurality of first internal data each generated by the first generation process for each piece of the first feature data and a second learning parameter;
   a reallocation process reallocating each piece of the first feature data into a transform destination position in the feature space on a basis of a first transform result in time series by the first transform process for each piece of the first internal data and the first feature data groups;
   a first calculation process calculating a first predicted value corresponding to the first feature data groups on a basis of a reallocation result by the reallocation process and a third learning parameter;
   an optimization process optimizing the first learning parameter, the second learning parameter, and the third learning parameter by statistical gradient on a basis of the response variable and the first predicted value calculated by the first calculation process;
   a second generation process generating second internal data based on time of one piece of second feature data among plural pieces of the second feature data each containing a plurality of features, the second internal data being generated for each piece of the second feature data on a basis of second feature data groups in each of which the plural pieces of the second feature data each containing the plurality of features are present in time series, a second internal parameter that is at least part of other piece of the second feature data at time before the time of the one piece of the second feature data, and a first learning parameter optimized by the optimization process;
   a second transform process transforming a position of the one piece of the second feature data in the feature space on a basis of a plurality of second internal data generated by the second generation process for each piece of the second feature data and a second learning parameter optimized by the optimization process; and
   an importance calculation process calculating importance data indicating an importance of each piece of the second feature data on a basis of a second transform result in time series by the second transform process for each piece of the second internal data and a third learning parameter optimized by the optimization process.

9. A time series data analysis program for a processor capable of accessing a database, the database storing a training data set having a predetermined number of first feature data groups in each of which plural pieces of first feature data each containing a plurality of features are present in time series and a predetermined number of response variables each corresponding to each piece of the first feature data in each of the first feature data groups, the program for the processor, comprising:

executing a first generation process generating first internal data based on time of one piece of the first feature data for each piece of the first feature data on a basis of the first feature data groups, a first internal parameter that is at least part of other piece of the first feature data at time before the time of the one piece of the first feature data, and a first learning parameter;

executing a first transform process transforming a position of the one piece of the first feature data in a feature space on the basis of a plurality of first internal data each generated by the first generation process for each piece of the first feature data and a second learning parameter;

executing a reallocation process reallocating each piece of the first feature data into a transform destination position in the feature space on a basis of a first transform result in time series by the first transform process for each piece of the first internal data and the first feature data groups;

executing a first calculation process calculating a first predicted value corresponding to the first feature data groups on a basis of a reallocation result by the reallocation process and a third learning parameter;

executing an optimization process optimizing the first learning parameter, the second learning parameter, and the third learning parameter by statistical gradient on a basis of the response variable and the first predicted value calculated by the first calculation process;

executing a second generation process generating second internal data based on time of one piece of second feature data among plural pieces of the second feature data each containing a plurality of features, the second feature data being generated for each piece of the second feature data on a basis of second feature data groups in each of which the plural pieces of the second feature data each containing the plurality of features are present in time series, a second internal parameter that is at least part of other piece of the second feature data at time before the time of the one piece of the second feature data, and a first learning parameter optimized by the optimization process;

executing a second transform process transforming a position of the one piece of the second feature data in the feature space on a basis of a plurality of second internal data generated by the second generation process for each piece of the second feature data and a second learning parameter optimized by the optimization process; and

executing an importance calculation process calculating importance data indicating an importance of each piece of the second feature data on a basis of a second transform result in time series by the second transform process for each piece of the second internal data and a third learning parameter optimized by the optimization process.

FIG.1

# FIG.2A

- 205 MONITOR
- 204 INPUT DEVICE
- 203 PROCESSOR
- 202 MEMORY
- 201 HDD
- 200

- 210 (network cloud)

- 225 MONITOR
- 224 INPUT DEVICE
- 223 PROCESSOR
- 222 MEMORY
- 221 HDD
- 220

2

# FIG.2B

- 200
- 251
- 252 TEST DATA SET
- 253 PREDICTION RESULT

- 210 (network cloud)

- 261 LEARNING SECTION
- 262 PREDICTION SECTION
- 220
- 263 TRAINING DATA SET
- 264
- 265 LEARNING PARAMETER

FIG.3

EP 3 624 017 A1

# FIG.4

START

S401 READ TRAINING DATA SET

LEARNING PHASE — S402 LEARNING PARAMETER GENERATION PROCESS

PREDICTION PHASE — S403 READ TEST DATA SET

S404 CALCULATE IMPORTANCE OF FEATURE

S405 CALCULATE PREDICTED VALUE

S406 STORE PREDICTED VALUE AND IMPORTANCE

S407 OUTPUT RESULT

END

S421 CALCULATE INTERNAL VECTOR

S422 CALCULATE TRANSFORM VECTOR

S423 CALCULATE REALLOCATION VECTOR

S424 CALCULATE PREDICTED VALUE

S425 OPTIMIZE LEARNING PARAMETER

S426 STORE LEARNING PARAMETER

EP 3 624 017 A1

# FIG. 5

EP 3 624 017 A1

# FIG.6

600

**Featue Importance**

Probability
57%

$x_1$ : 15%
$x_2$ : 14%
$x_3$ : 6%
$x_4$ : 5%
$x_5$ : 4%
$x_6$ : 4%
$x_7$ : 4%
$x_8$ : 3%
$x_9$ : 2%

One of the features, $x_4$, raises *Probability* by 5%. Compared with $x_1$, $x_2$, and $x_3$, contribution to *Probability* is small.

low high

EP 3 624 017 A1

# FIG.7

700

| TECHNIQUE | AUC |
|---|---|
| DISCRIMINATOR OF Transformer (NON-PATENT DOCUMENT 4) | 0.783 ± 0.027 |
| TIME SERIES DATA ANALYSIS APPARATUS 220 ACCORDING TO FIRST EMBODIMENT | 0.790 ± 0.054 |

Europäisches
Patentamt

European
Patent Office

Office européen
des brevets

## EUROPEAN SEARCH REPORT

Application Number

EP 19 19 4591

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/158552 A1 (LIU YAN [US] ET AL) 7 June 2018 (2018-06-07)<br>* figures 1-3,9-15 *<br>* paragraph [0003] - paragraph [0007] *<br>* paragraph [0026] *<br>* paragraph [0031] - paragraph [0057] *<br>* paragraph [0099] - paragraph [0101] *<br>----- | 1-9 | INV.<br>G06N3/04<br>G06N5/04<br>G06N3/08 |
| A | US 2017/032241 A1 (CORRADO GREGORY SEAN [US] ET AL) 2 February 2017 (2017-02-02)<br>* figures 1,3,4,7 *<br>* paragraph [0003] - paragraph [0009] *<br>* paragraph [0023] - paragraph [0031] *<br>* paragraph [0036] - paragraph [0046] *<br>* paragraph [0055] - paragraph [0058] *<br>* paragraph [0061] *<br>* paragraph [0067] *<br>* paragraph [0073] - paragraph [0075] *<br>----- | 1-9 | |

TECHNICAL FIELDS
SEARCHED (IPC)

G06N
G16H

The present search report has been drawn up for all claims

| Place of search | Date of completion of the search | Examiner |
|---|---|---|
| The Hague | 29 January 2020 | De Meyer, Arnaud |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...........................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 19 4591

This annex lists the patent family members relating to the patent documents cited in the above-mentioned European search report.
The members are as contained in the European Patent Office EDP file on
The European Patent Office is in no way liable for these particulars which are merely given for the purpose of information.

29-01-2020

| Patent document cited in search report | | Publication date | Patent family member(s) | | Publication date |
|---|---|---|---|---|---|
| US 2018158552 | A1 | 07-06-2018 | NONE | | |
| US 2017032241 | A1 | 02-02-2017 | CN | 107995992 A | 04-05-2018 |
| | | | EP | 3274887 A1 | 31-01-2018 |
| | | | JP | 6530084 B2 | 12-06-2019 |
| | | | JP | 2018526697 A | 13-09-2018 |
| | | | KR | 20170132842 A | 04-12-2017 |
| | | | US | 2017032241 A1 | 02-02-2017 |
| | | | WO | 2017019706 A1 | 02-02-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018170769 A

**Non-patent literature cited in the description**

- Why should I trust you?: Explaining the predictions of any classifier. **RIBEIRO ; MARCO TULIO ; SAMEER SINGH ; CARLOS GUESTRIN.** Proceedings of the 22nd ACM SIGKDD International Conference on Knowledge Discovery and Data Mining. ACM, 2016
- **FRIEDMAN J ; TREVOR H ; ROBERT T.** The elements of statistical learning. Springer, 2001, 119
- **GOLAS, SARA BERSCHE et al.** A machine learning model to predict the risk of 30-day readmissions in patients with heart failure: a retrospective analysis of electronic medical records data. *BMC medical informatics and decision making 18.1,* 22 June 2018, vol. 44
- **ASHISH VASWANI et al.** Attention is all you need. *Advances in Neural Information Processing Systems,* 2017